(19) Europäisches Patentamt
European Patent Office
Office européen des brevets

(11) Publication number : **0 588 750 A2**

# (12) EUROPEAN PATENT APPLICATION

(21) Application number : **93500120.6**

(22) Date of filing : **05.08.93**

(51) Int. Cl.[5] : **C12N 15/49,** A61K 39/21, G01N 33/569, C12P 21/08, A61K 39/395, C12N 5/20

The applicant has subsequently filed a sequence listing and declared, that it includes no new matter.

(30) Priority : **05.08.92 CU 8992**

(43) Date of publication of application : **23.03.94 Bulletin 94/12**

(84) Designated Contracting States : **AT BE CH DE DK ES FR GB GR IE IT LI LU MC NL PT SE**

(71) Applicant : **CENTRO DE INGENIERIA GENETICA Y BIOTECNOLOGIA
Avenida 31, Cubanacan Playa
Havana (CU)**

(72) Inventor : **Duarte Cano, Carlos A.
Ave. 31 No. 18307 Apto.12, entre 152 y 154
Playa, Ciudad Habana (CU)**
Inventor : **Menéndez Alarcon, Alfredo
Calle 184, No.3112 Apto,51/P-14
Playa, Ciudad Habana (CU)**
Inventor : **Montero Ramos, Marinieve
Calle 184, No.3112 Apto,51/P-14
Playa, Ciudad Habana (CU)**
Inventor : **Padron Palomares, Gabriel Ramon
Ave.31, No.18307, Apto.18
Playa, Ciudad Habana (CU)**
Inventor : **Herrera Matinez, Luis Saturnino
Calle 96, No. 306
Playa, Ciudad Habana (CU)**
Inventor : **Rodriguez Orta, Maria del Carmen
Calle 13, No. 2016
Matanzas (CU)**

(74) Representative : **Gil-Vega, Victor
Estébanez Calderon, 3 - 5B
E-28020 Madrid (ES)**

## (54) Method for the production of recombinant polypeptides bearing epitopes from different hiv isolates, and their uses as immunogens and in the detection of antibodies against hiv.

(57) The present invention provides recombinant polypeptides bearing selected epitopes from Human Immunodeficiency Virus (HIV) isolates.

The technical aim is to design and obtain, by means of chemical synthesis, genes encoding for a polypeptide composed not only by different HIV epitopes but also including epitopes corresponding to different HIV isolates. Said polypeptides correspond to the general formulae :

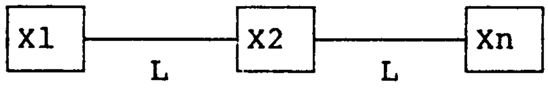


Where :

I. X1 to Xn represent fragments or viral proteins capable of inducing neutralizing antibodies or peptidic fragments capable of stimulating the proliferation and activation of helper T lymphocytes.

II. L represents spacer sequence or "linkers" located between two X regions.

Said polypeptides are capable of inducing neutralizing antibodies against HIV. Said polypeptides or antibodies generated against it could be useful for the diagnosis, treatment or prevention of AIDS.

EP 0 588 750 A2

**Background of the Invention:**

The Human Immunodeficiency Virus (HIV) is the etiological agent of the Acquired Immunodeficiency Syndrome (AIDS). (Popovic, M.; Sarngadharan, M.; Read, G., and Gallo, R.C. (1984), Science 224:497-500). It is known that HIV is able to infect not only T4+ lymphocytes as originally thought (Klatzman, D.; Barre Sinoussi, F.; Nugeyre, M.T.; Dauguet, C.; Vilmer, E.; Griscelli, C.; Brun-Vezinet, F.; Rouzioux, C.; Gluckman, J.D.; Chermann, J.C. and Montagnier, L. (1984), Science 225:59-63), but also other cell types as macrophages, and nervous and epithelial cells.

The HIV infection remains in a latent state during a long incubation period despite the fact that high levels of antibodies are maintained in the serum. This hidden infection causes a progressive deterioration of the immune system, with the reduction of T4 lymphocytes as the hallmark of the disease. The organism deprived of its principal line of defense becomes the target of several opportunistic infections which finally cause its death.

Due to the large magnitude of the AIDS epidemic and the lack of an adequate treatment, there is an urgent need in the world to develop an effective vaccine for the prevention of the disease.

The HIV virus has several characteristics which make this task extremely complex. The most troublesome is the high variability exhibited by its proteins, specially the external envelope glycoprotein gp120, which displays the main domains involved in its infectivity and constitutes the primary target for viral neutralization.

The immunization of primates with recombinant proteins (Berman, P.W.; Gregory T.J.; Lavon, R.; Nakamura, G.R.; Champe, M.A.; Porter, J.P.; Wurm F.M.; Hershberg, R.D.; Cobb, G.K. and Eichberg, J.W. 1990. Nature, Vol. 345:622-625. Girard, M.; Kieny, M.P.; Pinter A.; Barre-Sinoussi F.; Nara, P.; Kolbe, H.; Kusumi K.; Chaput A.; Rainhart T.; Muchmore, E.; Ronco, J.; Kaczorek, M.; Gomard, E.; Gluckman, J.C. and Fultz P.N., (1991). PNAS 88:542-546) has demonstrated the feasibility of generating protective immune response against HIV. However, in those experiments, the viral challenge has been developed in ideal conditions, in which the route, doses and immunization regime were very far from what we could expect to find in the natural infection. Not less important is the fact that it remains to be demonstrated if these immunogens can provide protection against a challenge with a divergent viral isolate.

Different vaccine candidates are presently been investigated in phase 1 and 2 clinical trials (Spalding, B.J. 1992, Biothecnology, 10:25-29). The vast majority of these candidates are based on the viral glycoproteins: gp160 (MicroGeneSys, Immuno) and gp120 (Genentech, Chiron). According to the available information of anti HIV immune response, the vaccination with these recombinant proteins does not guarantee protection against multiple HIV isolates. On the other hand the data available from first trials in humans with those vaccine candidates shows that only discrete levels of antibodies have been elicited. (Redfield, R.R.; Birx, D.I.; Ketter, N.; Tramont, E.; Polonis, E.; Davis, C.; Brundage, J.F.; Smith, G.; Johnson, S.; Fowler, A. 1991. New Eng. Jour. Med. 324 (24):1677-1684. Dolin, R.; Graham, B.S.; Greenberg, S.B.; Tacket, C.O.; Belshe, R.B.; Midthun, K.; Clements, M.L.; Gorse, G.J.; Horgan, G.R.L. Atmar (1991). Ann. Inter. Med. 114 (2):119-127).

The domain known as the Principal Neutralization Domain (PND) has been located in the third hypervariable region (V3) of gp120 (Rusche, J.R.; Javaherian, K.; McDanal, C.; Petro, J.; Lynn, Fischinger, P.J., Bolognesi, D.P., Putney, S.D. and Matthews, T.J. 1988. Proc. Natl. Acad. Sci. USA, 85:3198-3202. PNAS 85:4478-4482, 1988. Javaherian, K.; Langlois, A.J.; McDanal, C.; Ross, K.L.; Eckler, L.I.; Jellis, C.L.; Profy, A.T.; Rusche, J.R.; Bolognesi, D.P.; Putney, S.D. and Matthews, T.J. 1989. Proc. Natl. Acad. Sci. USA, 86:6768-6772). This region forms an exposed loop defined by two conserved cysteines in positions 303-309. (Palker, T.J.; Clark, M.; Langlois, A.J.; Matthews, T.J.; Weinhold, K.J.; Randall, R.R.; Bolognesi, D.; Haynes, B.F. (1988). PNAS, 85:1932-1936. Rushe, J.R.; Javaherian, K.; McDanal, C.; Petro, J.; Lynn, D.L.; Grimaila, R.; Langlois, A.; Gallo, R.C.; Arthur, L.O.; Fischinger, P.J.; Bolognesi, D.P.; Putney, S.D.; Matthews, T.J. (1988). PNAS 83:3192-3202).

Its aminoacid variability has been extensively characterized in the recent years (La Rosa, G.J.; Davide J.P.; Weinhold, K.; Waterbury, J.A.; Profy, A.T.; Lewis, J.A.; Langlois, A.J.; Dreesman, G.R.; Boswell, R.N.; Shadduck, P.; Holley, L.H.; Karplus, M.; Bolognesi, D.P.; Matthews, T.J.; Emini, E.A.; Putney, S.D. (1990). Science, 249:932-925).

It has been demonstrated that animals immunized with synthetic peptides from this region develop antibodies which neutralize the HIV infectivity in vitro. This neutralizing response is mainly type specific because it is limited to those HIV isolates having V3 regions homologous to the immunogen employed. It has been suggested that a mixture or "cocktail" of synthetic peptides from this region could be used as immunogen against AIDS (Palker, T.J.; Matthews, T.J.; Langlois, A.; Tanner, M.E.; Martin, M.E.; Searce, R.M.; Kim, J.S.; Berzofsky, J.A.; Bolognesi, D.P.; and Haynes, B. (1989). The Journal of Immunology, 142, 10:3612-3619).

The main drawback to the use of synthetic peptides as vaccines is their lack of immunogenicity per se, requiring their coupling by chemical methods to a carrier protein in order to induce an antibody response.

The production of a multi-epitope polypeptide (MEP) has been recently described (Kumar, V.V.J.; Bansal, K.V.S.; Rao, S.J. (1992). Gene, 110:137-144), including different important epitopes from the Hepatitis B surface proteins.

**Summary of the invention**

The present invention provides recombinant polypeptides bearing selected epitopes from Human Immunodeficiency Virus (HIV) isolates.

The technical aim is to design and obtain, by means of chemical synthesis, genes encoding for a polypeptide composed not only by different HIV epitopes but also including epitopes corresponding to different HIV isolates. Said polypeptides correspond to the general formulae:

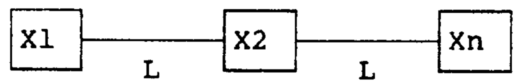


Where:

I. X1 to Xn represent fragments of viral proteins capable of inducing neutralizing antibodies or peptidic fragments capable of stimulating the proliferation and activation of helper T lymphocytes.

II. L represents spacer sequence or "linkers" located between two X regions.

Said polypeptides are capable of inducing neutralizing antibodies against HIV. Said polypeptides or antibodies generated against it could be useful for the diagnosis, treatment or prevention of AIDS.

**DETAILED DESCRIPTION OF THE INVENTION**

The present invention consists of the design and chemical synthesis of genes having the information for the production of proteins composed of different regions of the HIV which possess immunological relevance in the generation of a humoral or a cellular immune response against HIV. These regions are linked by spacer peptides composed of neutral aminoacids. Monoclonal antibodies, displaying anti HIV neutralizing activity generated against these proteins are also included in the present invention.

Additionally this kind of proteins can be successfully employed to develop serological methods for HIV diagnosis.

The essence of the present invention is the design and construction of synthetic genes coding for recombinant chimeric polypeptides bearing not only different epitopes from HIV but also epitopes corresponding to different viral isolates linked by spacer aminoacid sequences following the general formulae:

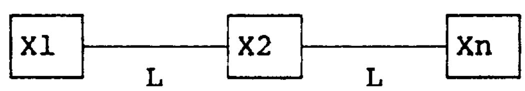


Where:

I. X1 to Xn represent fragments of viral proteins capable of inducing neutralizing antibodies or peptidic fragments capable of stimulating the proliferation and activation of helper T lymphocytes.

II. L represents spacer sequence or "linkers" located between two X regions.

More specifically, X represents the aminoacid sequence of the PND (V3) region of gp120 for different HIV isolates and the T cells epitopes from gp120 T1 and T2, while L represents the five aminoacid sequence ALA-GLY-GLY-GLY-ALA.

The polypeptides provided in the present invention are capable of eliciting antibodies which neutralize the infection of HIV in vitro to a permissive cell line.

The proteins of the subject invention or antibodies generated against it can be useful for the diagnosis, treatment or prevention of AIDS.

These genes, once synthesized, can be cloned in an appropriate expression vector for bacteria and produced intracellularly or as a product of secretion. Alternatively, it could be fused to a stabilizing DNA sequence, coding for an irrelevant fusion fragment, as in the present invention, for the purpose of enhancing the expression of the protein. Such a gene can be also cloned into vectors for its secretion or intracellular expression in yeast. These genes can also be inserted into a suitable vector for the expression of the protein, together with the adequate signal peptide, in mammalian or insect cells.

Independently of the host employed for its expression, the gene could be ligated after itself several times,

to generate repetitive polypeptides of higher molecular weight and augmented immunogenic potential.

The gene could also be fused to other genes, the product of which are able to form particles in eucariotic cells, for example PreS1-PreS2-S genes of Hepatitis B virus, gene C from the same virus or the retrotramposon Ty of yeast.

The proteins provided by this invention could form part of a vaccine preparation including, likewise other proteins or synthetic peptides from HIV as inducers of either T cell cytotoxic response or a B cell response of other nature.

The present invention provides also the hybridoma cell lines secreting the monoclonal antibodies (Mabs) 2C4 and 2F10 generated against one of the proteins provided herein. These Mabs recognizes with maximal affinity an epitope within the sequence RIHICPGRAFYT of the Principal Neutralization Domain of gp120 and reacts with V3 peptides from isolates MN, WMJI, WMJIII, SC and SF2. It neutralizes the HIV infection in vitro and therefore could be useful in the passive immunotherapy of individuals infected with or exposed to HIV infection.

The essence of the present invention is detailed through the following examples.

**EXAMPLE 1:**

The synthesis of the gene was performed according to well described methodologies (Jiménez, V.; Güimil, R.; Ubieta, R.; Ochagavia, M.; Silva, A.; Villegas, G. y Herrera, L. (1990). Biotecnología Aplicada 7,142-152). Different oligonucleotides were synthesized in an automated Gene Assembler Plus (Pharmacia) using Beta Cyanoethyl phosphoramidite chemistry. They were purified, phosphorylated and ligated as described.

The gen tab1 was designed to contain the information for a fifteen aminoacid epitope from the first constant region of gp120 (C1) (Modrow, S.; Hahn, B.H.; Shaw, G.M.; Gallo, R.C.; Wong-Staal, F. and Wolf, H. (1987). J. Virol., 61:570-578). It contains also the sequence for the 15 aminoacid of the tip of the V3 loop from the viral isolates MN, SC (Wong-Staal, F.; Shaw, G.M.; Hahn, B.H.; Salahuddin, S.Z.; Popovic, M.; Markham, P.; Redfield, R. and Gallo, R.C. (1985). Science 229:759-762. Gurgo, C.; Guo H.G.; Franchini, G.; Aldovini, A.; Collalti, E.; Farrell, K.; Wong-Staal, F.; Galli, R.C. and Reitz, Jr., M.S. (1988). Virology 164:531-536) and WMJII (Hahn, B.H.; Shaw, G.M.; Taylor, M.E.; Redfield, R.R.; Markham, P.D.; Salahuddin, S.Z.; Wong-Staal, F.; Gallo, R.C.; Parks, E.S. and Parks, W.P. 1986. Science 232, 1548-1553). These sequences were joined by DNA segments encoding for the five aminoacid linker AlaGlyGlyGlyAla.

**The base sequence of this gene is presented with number 1 in the list of sequences.**

Gene tab3 was synthesized employing the procedures already described. This gene includes the information for the V3 region of six HIV isolates: LR150 (isolate no. 150 from the list of La Rosa et al., 1990), JY1, RF, MN, BRVA, and IIIB. The same linker was used to join two neighboring epitopes. A NdeI restriction site was placed immediately before the stop codon in order to facilitate further manipulations. **The sequence of this gene is identified with number 7 in the sequence listing.**

The DNA fragment ts bears the information for two T helper-cells epitopes, T1 and T2 from gp120 (Berzofsky, J.; Bensassan, A.; Cease, K.; Bourge, J.; Cheynier, R.; Lurhuma, Z.; Salaun, J.; Gallo, R.; Shearer, G. and Zagury, D., (1988). Nature, 334:706). It was synthesized and cloned downstring of the gene tab3 to originate the gene tab5. **The DNA sequence of tab5 appears with number 9 on the sequence listing.**

The gene tab6 (**number 11 in the sequence listing**) was created by joining the fragment tc formed essentially by the same two epitopes T1 and T2, but it includes some additional aminoacids from the original sequence in the gp120 gene at both extremes of each epitope.

**EXAMPLE 2:**

Plasmid pT120S was constructed from vector pFP-15, previously described in detail (Novoa, L.I.; Madrazo, J.A.; Fernández, J.R.; Benítez, J.; Narciandi, E.; Rodríguez, J.L.; Estrada, M.P.; García, J. and Herrera, L. 1991. European Patent Application 416673 A1, Bulletin 91/11).

The vector pFP-15 bears the information for the first 62 aminoacids from the N terminal part of human interleukin-2 (IL-2) placed under the tryptophane promoter, and the coding sequence or ampicillin resistance.

This plasmid was restricted ClaI-BamHI and the large fragment was purified and ligated with the synthetic gene carrying cohesive ends ClaI and BglII. The resulting plasmid was named pT120S. The vector pFP-15 was also cleaved with XbaI, and the ends filled-in with Klenow enzyme. This preparation was further redigested with HindIII and the large fragment isolated by 0.8 % LGT agarose electrophoresis. Then, it was ligated with the fragment carrying the synthetic gene and terminator from pT120S, generated by means of a ClaI-Klenow-HindIII treatment. The resulting plasmid was called pITtab1.

This construct was employed to transform E. coli cells, MC1061 strain was used for plasmid propagation

and W3110 strain for expression.

Transformed cells were grown for 12 hours at 37ºC in LB medium supplemented by 50 μg/ml of ampicillin and 100 μg/ml of tryptophane. Cells were then transferred to M9 medium (sambrook, J., Fritsch, E.F. and Maniatis, T. (1989). Molecular Cloning: A Laboratory Manual, Cold Spring Harbor Laboratory Press) supplemented with 2 % casein hydrolyzate, 0.4 % glucose and 50 μg/ml of ampicillin with and initial OD (550 nm) or 0.05. After two hours β-indolacrilic acid was added for a final concentration of 20 μg/ml and was grown for two additional hours at 37º C.

The culture was centrifuged for 10 min. at 10 000 rpm and 20 μg of total protein were dissolved in Laemmli sample buffer, incubated at 95ºC for five minutes and subjected to 15 % SDS PAGE. A 18 kD prominent band was observed in SDS-PAGE of samples from pITtab1 tranformed cells. In contrast, no similar band could be detected on negative control. Electron-microscopy studies revealed that the protein accumulates into inclusion bodies in the cytoplasm of the bacteria. This protein was called TAB1 and **its aminoacid sequence is identified with number 2 in the sequence listing**. In order to test if TAB1 could be recognized by anti-HIV-1 human antibodies, a western blot analysis was performed. Proteins from the acrylamide gel were electroblotted to a Schleicher and Schuell 0.45 μm nitrocellulose membrane. The membrane was treated for two hours with a 1:20 dilution of a pool of well characterized human anti HIV sera and washed three times with Tris buffer saline (TBS) + 0.05% Tween 20. The membrane was then incubated in two steps with anti human biotinylated goat antibodies and streptavidine peroxidase (Boheringer Manhein) for 30' each and then 10' with diaminobenzidine substrate (Sigma).

The results showed that the 18 kD product was specifically recognized by a pool of sera from HIV-1 infected individuals. No band was observed in lanes corresponding to negative control.

The bacterial pellet, recovered by centrifugation at 12.000 x g at 4ºC, was suspended in bpS (8g/l NaCl; 1g/l $NaHPO_4$; 0,26 g/l $KH_2PO_4$; 0,2 g/l KCl, pH 7,2) at 100 g/l and sonicated at 4ºC . The cellular debris was washed once with 0.5% NP-40 and twice with bpS. Inclusion bodies were solubilized with PBS + 0.5% NP40 and 8M urea. This solution was clarified by centrifugation under the same conditions.

The supernatant was filtrated through a 0.45 um filter and loaded in a C4 reverse phase column of 10x250 mm (J.T. Baker, USA) using HPLC equipment (Pharmacia/LKB, Sweden). A linear gradient from 10 to 60% acetonitrile in 0.1% trifluoroacetic acid was used for the elution. A flow rate of 2 ml/min was employed and the optical density at 226 nm was monitored.

The desired protein was obtained with more than 95% purity in one of the collected fractions.

**EXAMPLE 3:**

The antigenic properties of TAB1 protein were assessed by ELISA. Ninety-six-well polystyrene microplates (Maxisorp, NUNC) were coated with 100ul of TAB1 protein at 10 μg/ml for 3 hours at 37ºC. Serum samples were diluted 1:20 in PBS-0.05% Tween 20, and incubated for 30 min. at 37ºC in the plates. After three washes with PBS-tween 20, the reaction was developed using a goat antihuman IgG(h)-horseradish peroxidase conjugate. Plates were washed again three times and hydrogen peroxide and orthophenylenediamine (0.05%) were used to detect bound antibodies. All sera were previously confirmed to be HIV positive by western blot in the National Reference Laboratory for AIDS of Havana, Cuba. The results of these experiments are summarized on table 1.

Differences in the pattern of sera reactivity against TAB1 and the natural HIV-$1_{(Bru)}$ gp120 in WB were found. While TAB1 detected 95% of HIV positive sera, gp120 was only recognized by the 86% of them. All ten HIV negative sera were nonreactive against TAB1. These results suggest that the TAB1 protein can detect a wider spectrum of HIV+ sera than gp120 from a single viral isolate (BRU).

**EXAMPLE 4:**

Two rabbits (New Zealand White) were inoculated with 0.2 mg of TAB-1 adsorbed in aluminum hydroxide. Four boosters were made every 30 days.

The levels of specific antibodies in the rabbits sera were measured by ELISA. Maxisorp microplates (NUNC) were coated with 10 ug/ml of either TAB-1 or a set of synthetic peptides representing the V3 regions included in the TAB1 protein (MN, WMJII, SC). V3 peptides from other isolates were also included. Three nonrelevant peptides were used as negative control.

The results are shown on table 2. Rabbits developed a potent anti TAB1 antibody response with titers of $1/10^6$ meassured by ELISA. Part of this response was shown to be directed against the synthetic peptides included in TAB1 (MN, WMJII and SC), and also to crossreacts with other V3 peptides (strains HTLVIIIB, RF, and BRVA).

**EXAMPLE 5:**

The methodology to obtain mouse monoclonal antibodies is widely known in the state of the art. Two groups of BALB/c mice (n=5) were immunized subcutaneously, every 2 weeks with four doses of 50 µg of TAB1 either with Freunds Adjuvant or Aluminum Hydroxide (50 µg AlOH/mg of protein).

Mouse sera were tested by ELISA against TAB1, IL2-p24 or a 15 aa V3 peptide from MN isolate. All the immunized mice seroconverted with an antibody titer of 1:2000 against the TAB1 protein and 1:100 against the synthetic MN peptide. The spleen cells from the mouse which developed the higher titer against the MN peptide were fused with the myeloma X63P3Ag14, according to the procedure described elsewhere (Duarte, C.A.; Fernández de Cossio, M.E.; Sierra, G.; Pentón, E.; Agraz, A.; Furrazola, G. y Aguilera, A. (1987). Interferón y Biotecnología, Vol. 4. No. 3:221-232). Growing cultures were assayed for antibodies against TAB1 using an indirect ELISA, similar to the one described above for human sera but using anti mouse Ig-HRPO to develop the reaction. Culture supernatants were previously diluted 1:20 with 10 mM phosphate buffer, ph=6.8 + 0.5% tween 20 + 0.5M NaCl (SDB). Optical density values three times higher than the negative control (nonrelated Mab) were regarded as positive. Five of these positive cultures were cloned and recloned by limiting dilution.

Stable secreting anti-TAB1 clones were also tested by ELISA against a 15-aminoacid synthetic peptide from the V3 loop of the MN isolate and against another fusion protein IL2-GP41, which contains the same fragment from the human IL-2. Two among these five clones, 2C4 and 10F10, secrete antibodies against the MN synthetic peptides. The antibody subclasses were shown to be IgG2a and IgG1 respectively, determined by double radial immunodiffusion using typing sera (ICN Immunobiologicals). The recognition pattern of these two Mabs was extensively studied using synthetic peptides, and the affinity constant for each peptide in solid phase was measured following the procedure described by Beatty et al (Beatty, J.D.; Beatty, B.G.; Vlahos, W.C.; 1987). Jour. Immunol. Met., 100:173-179). As it can be appreciated on table 3, both Mabs displayed similar binding characteristics and react with different affinities against different V3 peptides. The maximal affinity (108M-1) was obtained for isolates MN, WMJIII, WMJI and SC. Strains WMJII y SF2 were also recognized, but with lower affinity. Other more divergent isolates were not detected by these Mabs.

The specificity of these reactions was confirmed by a competition assay in which the binding of the Mabs to the TAB1 protein in solid phase was inhibited in a dose-dependent manner by soluble peptides (table 4). The results of these experiments correlate well with the binding of Mabs to solid-phase peptides.

**EXAMPLE 6:**

The neutralizing activity of Mabs was measured in a classical neutralization assay. Several dilutions of the Mabs 2C4 and F58/H3 (18) in RPMI 1640 medium with 10% fetal calf serum were incubated in triplicate together with 0.5 MOI of IIIB, SF2 and MN isolates for one hour at 37ºC in 96-well flat-bottom microplates. $10^5$ Jurkat-tat cells were added to each well and incubated again for one hour at 37ºC. Plates were centrifuged and the medium discarded. Cells were washed twice and incubated with 200 µl of medium at 37ºC. On day 4, 100 µl of medium were changed. Viral production was estimated on day 7 by p24 ELISA. Syncytium formation was also evaluated. The serum from either rabbit was able to neutralize the HIV at a 1:160 dilution. For Mabs 2C4 it was determined that an antibody concentration of 0.6 µg/ml was able to inhibit P24 production by 50%.

**EXAMPLE 7:**

Plasmid pISL31 has been described before in the patent referred to in the example 1. This plasmid contains a segment coding for a 24-aminoacid polypeptide from the amino terminal of human IL-2 (short stabilizer) and the gene coding for the human insulin under the tryptophane promoter. This vector was digested with XbaI, treated with S1 nuclease and further digested with HindIII. The larger fragment was purified and used for the construction of plasmid pITCtab2.

**The sequence of the gene tab2 is described in the sequence listing under number 3.** This gene may also be constructed by chemical synthesis although in our case it was obtained from the vector pITtab1 described in example 1. This plasmid was cut with the restriction enzymes EcoRV and HindIII and the shorter fragment coding for the three V3 regions and the linkers was purified. This gene was ligated to the vector prepared as described above yielding a gene fused to the IL-2 fragment. The resulting plasmid was called pITCtab2 and codifies for protein TAB2 (**number 4 in the sequence listing**).

This construction was employed to transform E.coli cells, that were grown and induced as described in the example 2.

The culture was centrifuged for 10 min at 10 000 rpm and 20 µg of total protein was dissolved in Laemmli sample buffer, incubated at 95ºC for five minutes and subjected to 15 % SDS PAGE . A 14Kd protein band

with high expression level was observed after Coomassie-blue staining of the gel.

Proteins were electroblotted to nitrocellulose membranes as described before, and the antigenic properties of the expressed protein were confirmed by using the monoclonal antibody 2C4 described in example 6. The biotin-streptavidin system (Boheringer Manhein) was used to develop the reaction. The results showed that the 14kD product was specifically recognized by Mab 2C4. No band was observed in lanes corresponding to cultures of negative control when incubated with anti HIV-1 sera.

For the purification of TAB2 protein, the growth of the cells, disruption, washed pellet and reverse phase chromatography were made following the procedures described in example 3 for protein TAB1.

**EXAMPLE 8:**

For the construction of plasmid pGHT-3 and the expression in Saccharomyces cerevisiae of protein TAB-2L, plasmid pIT120 (described in example 2) was digested with the restriction enzyme HindIII, treated with S1 nuclease and further digested with EcoRV. The smaller fragment (210 bp) bearing the coding sequence for the three V3 regions and the linkers was purified by electrophoresis in a low melting agarose gel.

Plasmid pBS-61 is a cloning vector intended for coupling genes to a DNA sequence coding for a signal peptide for secretion in the yeast Saccharomyces cerevisiae. It contains the promoter of the gene of Glyceraldehyde 3-Phosphate Dehydrogenase (GAP), followed by the region coding for the pre-pro Alpha factor from the same yeast. It contains besides the termination signals from genes GAP and Alpha Factor in tandem. The vector has a unique cloning HindIII site between the pre-pro Alpha Factor and the GAP terminator. The nuclease treatment after the digestion allows the insertion of genes in the proper reading frame to obtain a fusion protein having the signal for secretion.

The vector prepared in this way was ligated with the band described above. One clone with the insert in the desired orientation and the appropriate restriction pattern was selected and called pBHT-1. The nucleotide sequence of the gene tab2L, which includes the sequence coding for the signal peptide, **is shown with number 5 on the sequence listing**.

Plasmid pBHT-1 was then digested with BamHI, and the shorter band, containing from GAP promoter to Alpha Factor terminator, was purified. This fragment was ligated to plasmid pGM21, previously digested with BamHI and treated with Alkaline Phosphatase. Plasmid pGM21 is a bifunctional vector that can be propagated and selected in bacteria but also has the signals for replication (2 $\mu$) and selection (URA5) in yeast. After these procedures, a clone named pGHT-3 was selected.

This genetic construction was used to transform the strain SEY 2202 (MAT$\alpha$, ura3-52, leu2-3, leu2-112, his3-519) of S. cerevisiae by the LiCl method (Spencer, J.F., Spencer, D.M. and Bruce, I.J. (1989). Yeast Genetics. A manual of methods. Springer- Ve Verlag Berlin Heidelberg). Several individuals were analyzed for the expression of the desired protein. They were grown for 24 hours in 5 ml of media GO (1.32 g $(NH_4)_2HPO_4$; 0.9 g of $(NH_4)_2SO_4$; 0.50 g of Urea in a liter of bi-distilled water), supplemented by glucose 2% and acid casein hydrolysate (OXOID) at 0.5%. These cultures were inoculated in 50 L of rich medium YP (Yeast extract 1%, Peptone 2%) supplemented with 2% of Glucose. It was cultured for 72 hours, sampling each hour, and 0.5% of glucose added in each time. The samples were centrifuged and the culture medium was assayed for the presence of protein by dot immunobinding. 100 $\mu$l of media were applied under vacuum and subsequently reacted with thr Mab 2C4 previously described. The streptavidine-biotin system was employed to develop the reaction. The expression of this protein was evidenced by a positive reaction in this assay while negative cultures did not show any signal in this system.

**The aminoacid sequence for the TAB-2L protein is shown with number 6 on the sequence listing.**

**EXAMPLE 9:**

For the construction of plasmid pUCTab3 and the expression of protein TAB4, the plasmid pISL-31 was digested with the enzymes XbaI and BamHI, and the largest fragment was purified. This fragment was ligated to the synthetic gene tab3. The resulting plasmid was called pICT3. pICT3 was digested HindIII-ScaI and the fragment bearing the promotor, the gene and the terminator was cloned into the vector pUC19 previously digested HindIII-ScaI. The resulting plasmid was denominated pUCtab3.

This construct was employed to transform E.coli cells that were grown and induced as described in example 2. The expression of the desired protein was also evidenced by electrophoresis and western blot as described above. This protein was called TAB4 **and its aminoacid sequence is identified with number 8 on the sequence listing**.

**EXAMPLE 10:**

For the construction of plasmid pUCtab5 and pUCtab6 and the expression of the proteins TAB5 and TAB6, plasmid pUCTab3 was digested NdeI - BamHI. The largest fragment was then purified and ligated with either the synthetic oligonucleotides ts or tc. The genes resulting from the fusion of these fragments with tab3 were called tab5 and tab6, respectively.

These constructs were employed to transform E.coli cells that were grown and induced as described in example 2. The expression of the desired proteins was also evidenced by electrophoresis and western blot as described above. These proteins were named TAB5 and TAB6 **and their aminoacid sequences are described with numbers 10 and 12 on the sequence listing.**

**TABLE 1. COMPARISON OF THE REACTIVITY OF SERA AGAINST TAB1 IN ELISA AND GP120 (BRU) IN WESTERN BLOT.**

| Characteristics | | TAB-1 (+) | TAB-1 (-) |
|---|---|---|---|
| p120 WB (+) | n = 20 | 19 (95%) | 1 |
| p120 WB (-) | n = 7 | 6 (86%) | 1 |
| HIV WB (+) | n = 27 | 25 (93%) | 2 |
| HIV WB (-) | n = 10 | 0 ( 0%) | 10 |

A total of 27 positive and 10 negative sera by western blot criteria were used (rows 1 and 2). 20 out of the 27 positive sera had anti gp120 antibodies (row 3) whereas the other 7 sera had not (row 4). Numbers in parenthesis represent the % of anti TAB1 positive reactions for each group.

**TABLE 2. PEPTIDE ELISA WITH SERA FROM RABBITS IMMUNIZED WITH TAB1.**

| Peptide | Rab 1 | Rab 2 |
|---|---|---|
| TAB1 | 2x106 | 2x106 |
| MN | 32000 | 16000 |
| WMJII | 8000 | 8000 |
| SC | 2000 | 2000 |
| IIIB | 16000 | 16000 |
| RF | 16000 | 8000 |
| BRVA | 8000 | 8000 |

Antibody titer against TAB1 and V3 sinthetic peptides in ELISA. Absorbance values two times higher than the negative control (hyperimmune unrelated rabbit serum) were regarded as positive.

TABLE 3. AFFINITY CONSTANT FOR THE BINDING OF MABS TO THE PND PEPTIDES.

| PEPTIDE | SEQUENCE | Ka($M^{-1}$) Mab 2C4 | Mab 10F10 |
|---|---|---|---|
| TAB-1 | RKRihigpgrafyTT | $5.8x10^8$ | $7.6x10^8$ |
| MN | KRihigpgrafyTTK (C) | $5.8x10^8$ | $5.0x10^8$ |
| WMJIII | RRihigpgrafyTGK (C) | $5.2x10^8$ | $3.4x10^8$ |
| WMJI | RHihigpgrafyTGE (C) | $4.7x10^8$ | $2.2x10^8$ |
| SC | RSihigpgrafVATG (C) | $8.6x10^7$ | $8.6x10^7$ |
| SF2 | KSiYigpgrafHTTG (C) | $6.5x10^6$ | $7.2x10^6$ |
| WMJII | RSLSigpgrafRTRE (C) | $3.1x10^5$ | $4.3x10^5$ |
| NY5 | KGiAigpgrTLVARE (C) | 0 | 0 |
| CDC4 | KRVTLgpgrVWYTTG (C) | 0 | 0 |
| Z3 | QSiRigpgKVfVAKG (C) | 0 | 0 |
| LAVMAL | RGihFgpgQaLyTTG (C) | 0 | 0 |
| ELI | QRTPigLgQSLyTTR (C) | 0 | 0 |
| HTLVIIIB | IRIQRgpgrafVTIG (C) | 0 | 0 |
| negative control * | | 0 | 0 |

* 15-aminoacid peptide from human chorionic gonadotrophin (HCG).

TABLE 4. REACTIVITY OF MABS AGAINST PND PEPTIDES IN SOLUTION.

| PEPTIDE | SEQUENCE | I-50 Mab 2C4 | Mab 10F10 |
|---|---|---|---|
| TAB-1 | RKRihigpgrafyTT | 0.33 | 0.67 |
| MN | KRihigpgrafyTTK (C) | 0.32 | 0.40 |
| WMJIII | RRihigpgrafyTGK (C) | 0.34 | 0.27 |
| WMJI | RHihigpgrafyTGE (C) | 1.38 | 1.40 |
| SC | RSihigpgrafVATG (C) | 3.00 | 3.00 |
| SF2 | KSiYigpgrafHTTG (C) | 10.00 | 5.80 |
| NY5 | KGiAigpgrTLVARE (C) | >20 | >20 |
| LH-WMJ2 | RSLSigpgrafRTRE (C) | >20 | >20 |
| CDC4 | KRVTLgpgrVWYTTG (C) | >20 | >20 |
| Z3 | QSiRigpgKVfVAKG (C) | N.D | N.D |
| LAVMAL | RGihFgpgQaLyTTG (C) | >20 | >20 |
| ELI | QRTPigLgQSLyTTR (C) | >20 | >20 |
| IIIB | IRIQRgpgrafVTIG (C) | >20 | >20 |
| negative control * | | >20 | >20 |

Values express the concentration of peptide (µg/ml) needed to reduce by 50% the binding of Mab to the TAB1 protein in solid phase.

* 15-aminoacid peptide from human chorionic gonadotrophin (HCG).

1

SEQUENCE LISTING

(1) GENERAL INFORMATION:

(i) APPLICANT:
(A) NAME: CENTRO DE INGENIERIA GENETICA Y BIOTECNOLOGIA
(B) STREET: AVENIDA 31, CUBANACAN, PLAYA
(C) CITY: HABANA
(E) COUNTRY: CUBA
(F) POSTAL CODE (ZIP): 0000

(ii) TITLE OF INVENTION: METHOD FOR THE PRODUCTION OF RECOMBINANT POLYPEPTIDES BEARING EPITOPES FROM DIFFERENT HIV ISOLATES, AND THEIR USES AS IMMUNOGENS AND IN THE DETECTION OF ANTIBODIES AGAINST HIV

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 432 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(v) FRAGMENT TYPE: internal

(vi) ORIGINAL SOURCE:
(A) ORGANISM: Human immunodeficiency virus type 1

(ix) FEATURE:
(A) NAME/KEY: CDS
(B) LOCATION: 1..432

(xi) SEQUENCE DESCRIPTION: SEQ ID NO: 1:

```
ATG GCA CCT ACT TCA AGT TCT ACA AAG AAA ACA CAG CTA CAA CTG GAG       48
Met Ala Pro Thr Ser Ser Ser Thr Lys Lys Thr Gln Leu Gln Leu Glu
  1               5                  10                  15
```

2

```
CAT TTA CTG CTG GAT TTA CAG ATG ATT TTG AAT GGA ATT AAT AAT TAC      96
His Leu Leu Leu Asp Leu Gln Met Ile Leu Asn Gly Ile Asn Asn Tyr
            20                  25                  30

AAG AAT CCC AAA CTC ACC AGG ATG CTC ACA TTT AAG TTT TAC ATG CCC     144
Lys Asn Pro Lys Leu Thr Arg Met Leu Thr Phe Lys Phe Tyr Met Pro
        35                  40                  45

AAG AAG GCC ACA GAA CTG AAA CAT CTT CAG TGT CTA GCG ATG GCT TCT     192
Lys Lys Ala Thr Glu Leu Lys His Leu Gln Cys Leu Ala Met Ala Ser
    50                  55                  60

GAC GCA AAG GCT TAT GAT ACT GAG GTT CAT ATC CCT CCC CCT CCC CCA     240
Asp Ala Lys Ala Tyr Asp Thr Glu Val His Ile Pro Pro Pro Pro Pro
65                  70                  75                  80

CGT AAA CGC ATC CAC ATT GGA CCT GGT CGT GCA TTT TAT ACT ACC GCT     288
Arg Lys Arg Ile His Ile Gly Pro Gly Arg Ala Phe Tyr Thr Thr Ala
                85                  90                  95

GGA GGT GGT GCA CGT CGC TCC TTG TCT ATC GGC CCT GGA CGT GCT TTC     336
Gly Gly Gly Ala Arg Arg Ser Leu Ser Ile Gly Pro Gly Arg Ala Phe
            100                 105                 110

CGT ACC CGC GCA GGC GGT GGC GCA ACA CGT TCA ATC CAC ATT GGA CCT     384
Arg Thr Arg Ala Gly Gly Gly Ala Thr Arg Ser Ile His Ile Gly Pro
        115                 120                 125

GGT CGT GCA TTT TAT ACT ACC GCT GGA GGT GGT GCA ACC ATG GTA TA      432
Gly Arg Ala Phe Tyr Thr Thr Ala Gly Gly Gly Ala Thr Met Val
    130                 135                 140
```

(2) INFORMATION FOR SEQ ID NO: 2:

(i) SEQUENCE CHARACTERISTICS:
(A) LENGTH: 143 amino acids
(B) TYPE: amino acid
(D) TOPOLOGY: linear

(ii) MOLECULE TYPE: protein

(xi) SEQUENCE DESCRIPTION: SEQ ID NO: 2:

```
Met Ala Pro Thr Ser Ser Ser Thr Lys Lys Thr Gln Leu Gln Leu Glu
1               5                   10                  15

His Leu Leu Leu Asp Leu Gln Met Ile Leu Asn Gly Ile Asn Asn Tyr
            20                  25                  30

Lys Asn Pro Lys Leu Thr Arg Met Leu Thr Phe Lys Phe Tyr Met Pro
        35                  40                  45
```

3

```
Lys Lys Ala Thr Glu Leu Lys His Leu Gln Cys Leu Ala Met Ala Ser
    50                  55                  60

Asp Ala Lys Ala Tyr Asp Thr Glu Val His Ile Pro Pro Pro Pro Pro
65                  70                  75                  80

Arg Lys Arg Ile His Ile Gly Pro Gly Arg Ala Phe Tyr Thr Thr Ala
                85                  90                  95

Gly Gly Gly Ala Arg Arg Ser Leu Ser Ile Gly Pro Gly Arg Ala Phe
            100                 105                 110

Arg Thr Arg Ala Gly Gly Gly Ala Thr Arg Ser Ile His Ile Gly Pro
        115                 120                 125

Gly Arg Ala Phe Tyr Thr Thr Ala Gly Gly Gly Ala Thr Met Val
    130                 135                 140
```

(2) INFORMATION FOR SEQ ID NO: 3:

(i) SEQUENCE CHARACTERISTICS:
(A) LENGTH: 285 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(v) FRAGMENT TYPE: internal

(vi) ORIGINAL SOURCE:
(A) ORGANISM: Human immunodeficiency virus type 1

(ix) FEATURE:
(A) NAME/KEY: CDS
(B) LOCATION: 1..285

(xi) SEQUENCE DESCRIPTION: SEQ ID NO: 3:

```
ATG GCA CCT ACT TCA AGT TCT ACA GCA CAG ACA CAG CTA CAA CTG GAG      48
Met Ala Pro Thr Ser Ser Ser Thr Ala Gln Thr Gln Leu Gln Leu Glu
 1               5                  10                  15

CAT TTA CTG CTG GAT TTA CAG ATC TTT ATC GCT GGC GGT GGC GCA CGT      96
His Leu Leu Leu Asp Leu Gln Ile Phe Ile Ala Gly Gly Gly Ala Arg
            20                  25                  30

AAA CGC ATC CAC ATT GGA CCT GGT CGT GCA TTT TAT ACT ACC GCT GGA     144
Lys Arg Ile His Ile Gly Pro Gly Arg Ala Phe Tyr Thr Thr Ala Gly
        35                  40                  45
```

4

```
GGT GGT GCA CGT CGC TCC TTG TCT ACT GGC CCT GGA CGT GCT TTC CGT       192
Gly Gly Ala Arg Arg Ser Leu Ser Thr Gly Pro Gly Arg Ala Phe Arg
     50                  55                  60

ACC CGC GCA GGC GGT GGC GCA ACA CGT TCA ATC CAC ATT GGA CCT GGT       240
Thr Arg Ala Gly Gly Gly Ala Thr Arg Ser Ile His Ile Gly Pro Gly
 65                  70                  75                  80

CGT GCA TTT TAT GCT ACC GCT GGA GGT GGT GCA ACC ATG GTA TA            285
Arg Ala Phe Tyr Ala Thr Ala Gly Gly Gly Ala Thr Met Val
                 85                  90                  95
```

(2) INFORMATION FOR SEQ ID NO: 4:

(i) SEQUENCE CHARACTERISTICS:
(A) LENGTH: 94 amino acids
(B) TYPE: amino acid
(D) TOPOLOGY: linear

(ii) MOLECULE TYPE: protein

(xi) SEQUENCE DESCRIPTION: SEQ ID NO: 4:

```
Met Ala Pro Thr Ser Ser Ser Thr Ala Gln Thr Gln Leu Gln Leu Glu
  1               5                  10                  15

His Leu Leu Leu Asp Leu Gln Ile Phe Ile Ala Gly Gly Gly Ala Arg
             20                  25                  30

Lys Arg Ile His Ile Gly Pro Gly Arg Ala Phe Tyr Thr Thr Ala Gly
         35                  40                  45

Gly Gly Ala Arg Arg Ser Leu Ser Thr Gly Pro Gly Arg Ala Phe Arg
     50                  55                  60

Thr Arg Ala Gly Gly Gly Ala Thr Arg Ser Ile His Ile Gly Pro Gly
 65                  70                  75                  80

Arg Ala Phe Tyr Ala Thr Ala Gly Gly Gly Ala Thr Met Val
                 85                  90
```

(2) INFORMATION FOR SEQ ID NO: 5:

(i) SEQUENCE CHARACTERISTICS:
(A) LENGTH: 465 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

5

(v) FRAGMENT TYPE: internal

(vi) ORIGINAL SOURCE:
(A) ORGANISM: Human immunodeficiency virus type 1

(ix) FEATURE:
(A) NAME/KEY: CDS
(B) LOCATION: 1..465

(xi) SEQUENCE DESCRIPTION: SEQ ID NO: 5:

```
ATG AGA TTT CCT TCA ATT TTT ACT GCA GTT TTA TTC GCA GCA TCC TCC       48
Met Arg Phe Pro Ser Ile Phe Thr Ala Val Leu Phe Ala Ala Ser Ser
  1               5                  10                  15

GCA TTA GCT GCT CCA GTC AAC ACT ACA ACA GAA GAT GAA ACG GCA CAA       96
Ala Leu Ala Ala Pro Val Asn Thr Thr Thr Glu Asp Glu Thr Ala Gln
             20                  25                  30

ATT CCG GCT GAA GCT GTC ATC GGT TAC TCA GAT TTA GAA GGG GAT TTC      144
Ile Pro Ala Glu Ala Val Ile Gly Tyr Ser Asp Leu Glu Gly Asp Phe
         35                  40                  45

GAT GTT GCT GTT TTG CCA TTT TCC AAC AGC ACA AAT AAC GGG TTA TTG      192
Asp Val Ala Val Leu Pro Phe Ser Asn Ser Thr Asn Asn Gly Leu Leu
     50                  55                  60

TTT ATA AAT ACT ACT ATT GCC AGC ATT GCT GCT AAA GAA GAA GGG GTA      240
Phe Ile Asn Thr Thr Ile Ala Ser Ile Ala Ala Lys Glu Glu Gly Val
 65                  70                  75                  80

TCT CTA GAT AAA AGA ATC GCT GGC GGT GGC GCA CGT AAA CGC ATC CAC      288
Ser Leu Asp Lys Arg Ile Ala Gly Gly Gly Ala Arg Lys Arg Ile His
                 85                  90                  95

ATT GGA CCT GGT CGT GCA TTT TAT ACT ACC GCT GGA GGT GGT GCA CGT      336
Ile Gly Pro Gly Arg Ala Phe Tyr Thr Thr Ala Gly Gly Gly Ala Arg
            100                 105                 110

CGC TCC TTG TCT ATC GGC CCT GGA CGT GCT TTC CGT ACC CGC GCA GGC      384
Arg Ser Leu Ser Ile Gly Pro Gly Arg Ala Phe Arg Thr Arg Ala Gly
        115                 120                 125

GGT GGC GCA ACA CGT TCA ATC CAC ATT GGA CCT GGT CGT GCA TTT TAT      432
Gly Gly Ala Thr Arg Ser Ile His Ile Gly Pro Gly Arg Ala Phe Tyr
    130                 135                 140

ACT ACC GCT GGA GGT GGT GCA ACC ATG GTA TA                           465
Thr Thr Ala Gly Gly Gly Ala Thr Met Val
145                 150                 155
```

6

(2) INFORMATION FOR SEQ ID NO: 6:

(i) SEQUENCE CHARACTERISTICS:
(A) LENGTH: 154 amino acids
(B) TYPE: amino acid
(D) TOPOLOGY: linear

(ii) MOLECULE TYPE: protein

(xi) SEQUENCE DESCRIPTION: SEQ ID NO: 6:

```
Met Arg Phe Pro Ser Ile Phe Thr Ala Val Leu Phe Ala Ala Ser Ser
 1               5                  10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
 65                 70                  75                  80

Ser Leu Asp Lys Arg Ile Ala Gly Gly Gly Ala Arg Lys Arg Ile His
                85                  90                  95

Ile Gly Pro Gly Arg Ala Phe Tyr Thr Thr Ala Gly Gly Gly Ala Arg
            100                 105                 110

Arg Ser Leu Ser Ile Gly Pro Gly Arg Ala Phe Arg Thr Arg Ala Gly
        115                 120                 125

Gly Gly Ala Thr Arg Ser Ile His Ile Gly Pro Gly Arg Ala Phe Tyr
    130                 135                 140

Thr Thr Ala Gly Gly Gly Ala Thr Met Val
145                 150
```

(2) INFORMATION FOR SEQ ID NO: 7:

(i) SEQUENCE CHARACTERISTICS:
(A) LENGTH: 436 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

7

(v) FRAGMENT TYPE: internal

(vi) ORIGINAL SOURCE:
(A) ORGANISM: Human immunodeficiency virus type 1

(ix) FEATURE:
(A) NAME/KEY: CDS
(B) LOCATION: 1..426

(xi) SEQUENCE DESCRIPTION: SEQ ID NO: 7:

```
ATG GCA CCT ACT TCA AGT TCT ACA GCA CAG ACA CAG CTA CAA CTG GAG       48
Met Ala Pro Thr Ser Ser Ser Thr Ala Gln Thr Gln Leu Gln Leu Glu
  1               5                  10                  15

CAT TTA CTG CTG GAT TTA CAG ATC TTT CTA GAC TCG AGA GGC ATT CGT       96
His Leu Leu Leu Asp Leu Gln Ile Phe Leu Asp Ser Arg Gly Ile Arg
             20                  25                  30

ATC GGC CCA GGT CGC GCA ATT TTA GCA ACA GCT GGC GGT GGC GCA CGT      144
Ile Gly Pro Gly Arg Ala Ile Leu Ala Thr Ala Gly Gly Gly Ala Arg
         35                  40                  45

CAA TCT ACC CCT ATT GGT TTA GGT CAG GCT CTG TAT ACG ACT GCC GGC      192
Gln Ser Thr Pro Ile Gly Leu Gly Gln Ala Leu Tyr Thr Thr Ala Gly
     50                  55                  60

GGT GGT GCG CGC AAA AGT ATC ACC AAG GGT CCA GGC CGC GTC AGT TAC      240
Gly Gly Ala Arg Lys Ser Ile Thr Lys Gly Pro Gly Arg Val Ser Tyr
 65                  70                  75                  80

GCC ACC GCG GGC GGC GGT GCC CGT AAG CGT ATC CAC ATT GGC CCA GGC      288
Ala Thr Ala Gly Gly Gly Ala Arg Lys Arg Ile His Ile Gly Pro Gly
                 85                  90                  95

CGT GCA TTC TAT ACT ACA GCA GGT GGT GGC GCA CGT AAA CGC ATC ACT      336
Arg Ala Phe Tyr Thr Thr Ala Gly Gly Gly Ala Arg Lys Arg Ile Thr
            100                 105                 110

ATG GGT CCT GGT CGC GTC TAT TAC ACG ACC GCT GGC GGC GGT GCT AGC      384
Met Gly Pro Gly Arg Val Tyr Tyr Thr Thr Ala Gly Gly Gly Ala Ser
        115                 120                 125

ATT CGC ATC CAA CGC GGC CCT GGT CGT GCA TTT GTG ACC ATA              426
Ile Arg Ile Gln Arg Gly Pro Gly Arg Ala Phe Val Thr Ile
    130                 135                 140

TGATAACGCG                                                           436
```

8

(2) INFORMATION FOR SEQ ID NO: 8:

(i) SEQUENCE CHARACTERISTICS:
(A) LENGTH: 142 amino acids
(B) TYPE: amino acid
(D) TOPOLOGY: linear

(ii) MOLECULE TYPE: protein

(xi) SEQUENCE DESCRIPTION: SEQ ID NO: 8:

```
Met Ala Pro Thr Ser Ser Ser Thr Ala Gln Thr Gln Leu Gln Leu Glu
 1               5                  10                  15

His Leu Leu Leu Asp Leu Gln Ile Phe Leu Asp Ser Arg Gly Ile Arg
            20                  25                  30

Ile Gly Pro Gly Arg Ala Ile Leu Ala Thr Ala Gly Gly Gly Ala Arg
        35                  40                  45

Gln Ser Thr Pro Ile Gly Leu Gly Gln Ala Leu Tyr Thr Thr Ala Gly
    50                  55                  60

Gly Gly Ala Arg Lys Ser Ile Thr Lys Gly Pro Gly Arg Val Ser Tyr
65                  70                  75                      80

Ala Thr Ala Gly Gly Gly Ala Arg Lys Arg Ile His Ile Gly Pro Gly
                85                  90                  95

Arg Ala Phe Tyr Thr Thr Ala Gly Gly Gly Ala Arg Lys Arg Ile Thr
            100                 105                 110

Met Gly Pro Gly Arg Val Tyr Tyr Thr Thr Ala Gly Gly Gly Ala Ser
        115                 120                 125

Ile Arg Ile Gln Arg Gly Pro Gly Arg Ala Phe Val Thr Ile
    130                 135                 140
```

(2) INFORMATION FOR SEQ ID NO: 9:

(i) SEQUENCE CHARACTERISTICS:
(A) LENGTH: 534 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(v) FRAGMENT TYPE: internal

(vi) ORIGINAL SOURCE:
(A) ORGANISM: Human immunodeficiency virus type 1

9

```
(ix) FEATURE:
      (A) NAME/KEY: CDS
      (B) LOCATION: 1..529

(xi) SEQUENCE DESCRIPTION: SEQ ID NO: 9:

ATG CCA CCT ACT TCA AGT TCT ACA GCA CAG ACA CAG CTA CAA CTG GAG        48
Met Pro Pro Thr Ser Ser Ser Thr Ala Gln Thr Gln Leu Gln Leu Glu
  1               5                  10                  15

CAT TTA CTG CTG GAT TTA CAG ATC TTT CTA GAC TCG AGA GGC ATT CGT        96
His Leu Leu Leu Asp Leu Gln Ile Phe Leu Asp Ser Arg Gly Ile Arg
             20                  25                  30

ATC GGC CCA GGT CGC GCA ATT TTA GCA ACA GCT GGC GGT GGC GCA CGT       144
Ile Gly Pro Gly Arg Ala Ile Leu Ala Thr Ala Gly Gly Gly Ala Arg
         35                  40                  45

CAA TCT ACC CCT ATT GGT TTA GGT CAG GCT CTG TAT ACG ACT GCC GGC       192
Gln Ser Thr Pro Ile Gly Leu Gly Gln Ala Leu Tyr Thr Thr Ala Gly
     50                  55                  60

GGT GGT GCG CGC AAA AGT ATC ACC AAG GGT CCA GGC CGC GTC ATT TAC       240
Gly Gly Ala Arg Lys Ser Ile Thr Lys Gly Pro Gly Arg Val Ile Tyr
 65                  70                  75                  80

GCC ACC GCG GGC GGC GGT GCC CGT AAG CGT ATC CAC ATT GGC CCA GGC       288
Ala Thr Ala Gly Gly Gly Ala Arg Lys Arg Ile His Ile Gly Pro Gly
                 85                  90                  95

CGT GCA TTC TAT ACT ACA GCA GGT GGT GGC GCA CGT AAA CGC ATC ACT       336
Arg Ala Phe Tyr Thr Thr Ala Gly Gly Gly Ala Arg Lys Arg Ile Thr
            100                 105                 110

ATG GGT CCT GGT CGC GTC TAT TAC ACG ACC GCT GGC GGC GGT GCT AGC       384
Met Gly Pro Gly Arg Val Tyr Tyr Thr Thr Ala Gly Gly Gly Ala Ser
        115                 120                 125

ATT CGC ATC CAA CGC GGC CCT GGT CGT GCA TTT GTG ACC ATA GCT GGA       432
Ile Arg Ile Gln Arg Gly Pro Gly Arg Ala Phe Val Thr Ile Ala Gly
    130                 135                 140

GGT GGT GCA AAA CAG ATT ATC AAC ATG TGG CAG GAA CTG GGC AAA GCG       480
Gly Gly Ala Lys Gln Ile Ile Asn Met Trp Gln Glu Leu Gly Lys Ala
145                 150                 155                 160

ATG TAT GCC CAT GAA GAT ATC ATT AGC CTG TGG GAC CAG TCT CTT AAG T     529
Met Tyr Ala His Glu Asp Ile Ile Ser Leu Trp Asp Gln Ser Leu Lys
                165                 170                 175

GATAA                                                                  534
```

10

(2) INFORMATION FOR SEQ ID NO: 10:

(i) SEQUENCE CHARACTERISTICS:
(A) LENGTH: 176 amino acids
(B) TYPE: amino acid
(D) TOPOLOGY: linear

(ii) MOLECULE TYPE: protein

(xi) SEQUENCE DESCRIPTION: SEQ ID NO: 10:

```
Met Pro Pro Thr Ser Ser Ser Thr Ala Gln Thr Gln Leu Gln Leu Glu
 1               5                  10                  15

His Leu Leu Leu Asp Leu Gln Ile Phe Leu Asp Ser Arg Gly Ile Arg
            20                  25                  30

Ile Gly Pro Gly Arg Ala Ile Leu Ala Thr Ala Gly Gly Gly Ala Arg
        35                  40                  45

Gln Ser Thr Pro Ile Gly Leu Gly Gln Ala Leu Tyr Thr Thr Ala Gly
    50                  55                  60

Gly Gly Ala Arg Lys Ser Ile Thr Lys Gly Pro Gly Arg Val Ile Tyr
65                  70                  75                  80

Ala Thr Ala Gly Gly Gly Ala Arg Lys Arg Ile His Ile Gly Pro Gly
                85                  90                  95

Arg Ala Phe Tyr Thr Thr Ala Gly Gly Gly Ala Arg Lys Arg Ile Thr
            100                 105                 110

Met Gly Pro Gly Arg Val Tyr Tyr Thr Thr Ala Gly Gly Gly Ala Ser
        115                 120                 125

Ile Arg Ile Gln Arg Gly Pro Gly Arg Ala Phe Val Thr Ile Ala Gly
    130                 135                 140

Gly Gly Ala Lys Gln Ile Ile Asn Met Trp Gln Glu Leu Gly Lys Ala
145                 150                 155                 160

Met Tyr Ala His Glu Asp Ile Ile Ser Leu Trp Asp Gln Ser Leu Lys
                165                 170                 175
```

(2) INFORMATION FOR SEQ ID NO: 11:

(i) SEQUENCE CHARACTERISTICS:
(A) LENGTH: 570 base pairs
(B) TYPE: nucleic acid
(C) STRANDEDNESS: double
(D) TOPOLOGY: linear

(ii) MOLECULE TYPE: DNA (genomic)

11

(iii) HYPOTHETICAL: NO

(v) FRAGMENT TYPE: internal

(vi) ORIGINAL SOURCE:
(A) ORGANISM: Human immunodeficiency virus type 1

(ix) FEATURE:
(A) NAME/KEY: CDS
(B) LOCATION: 1..563

(xi) SEQUENCE DESCRIPTION: SEQ ID NO: 11:

```
ATG GCA CCT ACT TCA AGT TCT ACA GCA CAG ACA CAG CTA CAA CTG GAG       48
Met Ala Pro Thr Ser Ser Ser Thr Ala Gln Thr Gln Leu Gln Leu Glu
  1               5                  10                  15

CAT TTA CTG CTG GAT TTA CAG ATC TTT CTA GAC TCG AGA GGC ATT CGT       96
His Leu Leu Leu Asp Leu Gln Ile Phe Leu Asp Ser Arg Gly Ile Arg
             20                  25                  30

ATC GGC CCA GGT CGC GCA ATT TTA GCA ACA GCT GGC GGT GGC GCA CGT      144
Ile Gly Pro Gly Arg Ala Ile Leu Ala Thr Ala Gly Gly Gly Ala Arg
         35                  40                  45

CAA TCT ACC CCT ATT GGT TTA GGT CAG GCT CTG TAT ACG ACT GCC GGC      192
Gln Ser Thr Pro Ile Gly Leu Gly Gln Ala Leu Tyr Thr Thr Ala Gly
     50                  55                  60

GGT GGT GCG CGC AAA AGT ATC ACC AAG GGT CCA GGC CGC GTC ATT TAC      240
Gly Gly Ala Arg Lys Ser Ile Thr Lys Gly Pro Gly Arg Val Ile Tyr
 65                  70                  75                  80

GCC ACC GCG GGC GCC GGT GCC CGT AAG CGT ATC CAC ATT GGC CCA GGC      288
Ala Thr Ala Gly Ala Gly Ala Arg Lys Arg Ile His Ile Gly Pro Gly
                 85                  90                  95

CGT GCA TTC TAT ACT ACA GCA GGT GGT GGC GCA CGT AAA CGC ATC ACT      336
Arg Ala Phe Tyr Thr Thr Ala Gly Gly Gly Ala Arg Lys Arg Ile Thr
            100                 105                 110

ATG GGT CCT GGT CGC GTC TAT TAC ACG ACC GCT GGC GGC GGT GCT AGC      384
Met Gly Pro Gly Arg Val Tyr Tyr Thr Thr Ala Gly Gly Gly Ala Ser
        115                 120                 125

ATT CGC ATC CAA CGC GGC CCT GGT CGT GCA TTT GTG ACC ATA GCT GGA      432
Ile Arg Ile Gln Arg Gly Pro Gly Arg Ala Phe Val Thr Ile Ala Gly
    130                 135                 140

GGT GGT GCA CGT ATC AAA CAG ATT ATC AAC ATG TGG CAG GAA GTG GGC      480
Gly Gly Ala Arg Ile Lys Gln Ile Ile Asn Met Trp Gln Glu Val Gly
145                 150                 155                 160
```

12

```
AAA GCG ATG TAT GCC CCG CCG ATT TCT GGT ATG GTT GAG CAG ATG CAT        528
Lys Ala Met Tyr Ala Pro Pro Ile Ser Gly Met Val Glu Gln Met His
                165                 170                 175

GAA GAT ATC ATT AGC CTG TGG GAC CAG TCT CTT AA GTGATAA                 570
Glu Asp Ile Ile Ser Leu Trp Asp Gln Ser Leu
            180                 185
```

(2) INFORMATION FOR SEQ ID NO: 12:

(i) SEQUENCE CHARACTERISTICS:
(A) LENGTH: 187 amino acids
(B) TYPE: amino acid
(D) TOPOLOGY: linear

(ii) MOLECULE TYPE: protein

(xi) SEQUENCE DESCRIPTION: SEQ ID NO: 12:

```
Met Ala Pro Thr Ser Ser Ser Thr Ala Gln Thr Gln Leu Gln Leu Glu
  1               5                  10                  15

His Leu Leu Leu Asp Leu Gln Ile Phe Leu Asp Ser Arg Gly Ile Arg
            20                  25                  30

Ile Gly Pro Gly Arg Ala Ile Leu Ala Thr Ala Gly Gly Gly Ala Arg
        35                  40                  45

Gln Ser Thr Pro Ile Gly Leu Gly Gln Ala Leu Tyr Thr Thr Ala Gly
    50                  55                  60

Gly Gly Ala Arg Lys Ser Ile Thr Lys Gly Pro Gly Arg Val Ile Tyr
 65                  70                  75                  80

Ala Thr Ala Gly Ala Gly Ala Arg Lys Arg Ile His Ile Gly Pro Gly
                 85                  90                  95

Arg Ala Phe Tyr Thr Thr Ala Gly Gly Gly Ala Arg Lys Arg Ile Thr
            100                 105                 110

Met Gly Pro Gly Arg Val Tyr Tyr Thr Thr Ala Gly Gly Gly Ala Ser
        115                 120                 125

Ile Arg Ile Gln Arg Gly Pro Gly Arg Ala Phe Val Thr Ile Ala Gly
    130                 135                 140

Gly Gly Ala Arg Ile Lys Gln Ile Ile Asn Met Trp Gln Glu Val Gly
145                 150                 155                 160

Lys Ala Met Tyr Ala Pro Pro Ile Ser Gly Met Val Glu Gln Met His
                165                 170                 175

Glu Asp Ile Ile Ser Leu Trp Asp Gln Ser Leu
            180                 185
```

## Claims

1. A method for the production of recombinant polypeptides containing epitopes from the Human Immunodeficiency Virus (HIV) which involves the design and construction of synthetic genes coding for chimeric polypeptides bearing not only different epitopes from HIV but more than one variant of the same epitope from different HIV isolates as well and the polypeptides are joined by linkers following the general formulae:

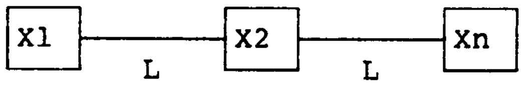


Where:
I. X1 to Xn represent fragments of viral proteins capable of inducing a neutralizing antibody response, or peptidic fragments that stimulate the proliferation and activation of helper T lymphocytes.
II. L represents spacer sequence or "linkers" located between two X regions. Being said polypeptides capable of inducing antibodies in immunized animals that neutralize the infectivity of HIV in vitro.

2. A method according to claim 1, wherein X represents sequences from the PND region from different HIV isolates and L is composed of the five-aminoacid sequence ALA-GLY-GLY-GLY-ALA.

3. A method, according to claim 1, wherein the synthetic genes are cloned into vectors for their expression intra or extracellularly in an appropriate host such as bacteria, yeast, mammalian or insect cells.

4. A method, according to claim 1, wherein the synthetic genes are fused to a sequence that stabilizes their expression, coding for fusion proteins.

5. DNA molecules obtained following essentially claims 1 to 4, whose nucleotide sequences are listed with numbers 1 to 6 on the sequence listing, coding for the proteins identified with numbers 7 to 12 on the sequence listing.

6. Chimeric polypeptides obtained following essentially the procedures claimed in claims 1 to 4, which are encoded by the DNA molecules refered to in claim 5, and whose aminoacid sequences are identified on the sequence listing with numbers 7 to 12.

7. Plasmids or recombinant vectors containing essentially some of the DNA molecules claimed in claim 5.

8. Plasmids or recombinant vectors as described in claim 7, where said plasmids correspond to pITtab1, pICTtab2, pUCTab3, pUCTab5, pUCTab6 and

9. Strains of recombinant microorganisms resulting from the transformation the appropriate host by any of the plasmids claimed in claim 8.

10. A vaccine composition containing any of the proteins claimed in claim 6, wherein such proteins are presented in a suitable excipient or coupled chemically or genetically to an appropriate carrier.

11. An assay for the detection of specific antibodies against the HIV virus in which one or several of the proteins claimed in claim 6 are used.

12. Hybridoma cell lines producing the monoclonal antibodies 2C4 and 2F10 generated against one of the proteins described in claim 7.

13. The monoclonal antibodies secreted by the hybridomas of claim 12 or fragments thereof, obtained either by chemical procedures or genetic engineering. Said antibodies recognize an epitope within the sequence RIHIGPGRAFYT of the PND of gp120 for HIV-1 and react, with the affinity constants described in the example 6, with the HIV isolated MN, WMJI, WMJIII, WMJII, SC y SF2, having neutralizing activity of the HIV in vitro.

14. The use of the monoclonal antibodies of claim 13 or fragments thereof in passive immunotherapy of individuals infected with the HIV or exposed to the virus.